(19) **Europäisches Patentamt**

**European Patent Office**

**Office européen des brevets**

(11) Veröffentlichungsnummer: **0 492 386 B1**

(12) **EUROPÄISCHE PATENTSCHRIFT**

(45) Veröffentlichungstag der Patentschrift: **07.12.94**

(51) Int. Cl.5: **C07C 19/08**, C07C 17/00, C07C 17/20, B01J 27/125

(21) Anmeldenummer: **91121681.0**

(22) Anmeldetag: **18.12.91**

(54) **Verfahren zur Herstellung von Fluor enthaltenden Ethanderivaten.**

(30) Priorität: **24.12.90 DE 4041771**

(43) Veröffentlichungstag der Anmeldung:
**01.07.92 Patentblatt 92/27**

(45) Bekanntmachung des Hinweises auf die Patenterteilung:
**07.12.94 Patentblatt 94/49**

(84) Benannte Vertragsstaaten:
**AT BE CH DE ES FR GB GR IT LI NL**

(56) Entgegenhaltungen:
**WO-A-89/11467**
**US-A- 2 767 227**
**US-A- 3 644 545**
**US-A- 4 088 704**

(73) Patentinhaber: **Kali-Chemie Aktiengesellschaft**
**Hans-Böckler-Allee 20**
**D-30173 Hannover (DE)**

(72) Erfinder: **Swidersky, Hans-Walter**
**Zeppelinstrasse 5**
**W-3000 Hannover 1 (DE)**
Erfinder: **Rudolph, Werner**
**Oderstrasse 38**
**W-3000 Hannover 38 (DE)**
Erfinder: **Born, Thomas**
**Über der Kirche 1**
**W-3201 Holle 5 (DE)**

(74) Vertreter: **Lauer, Dieter, Dr.**
**c/o Solvay Deutschland GmbH**
**Hans-Böckler-Allee 20**
**D-30173 Hannover (DE)**

EP 0 492 386 B1

Rank Xerox (UK) Business Services
(3.10/3.09/3.3.3)

EP 0 492 386 B1

**Beschreibung**

Die Erfindung bezieht sich auf ein Verfahren zur Herstellung von Fluor enthaltenden Ethanderivaten der allgemeinen Formel $CF_3\text{-}CHZ^1Z^2$, worin $Z^1$ und $Z^2$ gleich oder verschieden sein können und Wasserstoff, Fluor, Chlor oder Brom bedeuten können.

Fluor enthaltende Ethanderivate, welche mindestens 1 Wasserstoffatom aufweisen, beispielsweise $CF_3CH_3$ (R143a), $CF_3CH_2F$ (R134a), $CF_3CH_2Cl$ (R133a) oder $CF_3CHCl_2$ (R123), gelten als denkbare Ersatzstoffe für vollhalogenierte Fluorchlorkohlenwasserstoffe, da sie keine bzw. nur geringe Auswirkung auf die Ozonschicht haben. Sie können als Kältemittel, Lösungsmittel oder Treibmittel eingesetzt werden. Darüber hinaus sind diese Verbindungen auch in der chemischen Synthese wichtig. $CF_3CH_2Cl$ beispielsweise kann, wie in der europäischen Patentanmeldung EP-A 171 248 beschrieben wird, mit einem Karbonsäuresalz und Wasser zu 2,2,2-Trifluorethanol hydrolysiert werden. 2,2,2-Trifluorethanol wiederum ist ein Zwischenprodukt, beispielsweise bei der Herstellung des Anästhetikums Isofluorane.

Es ist bereits bekannt durch katalysierten Halogen-Fluor-Austausch, insbesondere durch Chlor-Fluor-Austausch, aus entsprechend halogenierten Derivaten oder ungesättigten Vorläufern solche halogenierten Derivate in der Gasphase herzustellen. In der US-A 2 885 427 wird die Herstellung von $CF_3CH_2Cl$ aus Trichlorethylen und Fluorwasserstoff in der Gasphase in Anwesenheit eines Chromfluorid-Katalysators beschrieben. Die Verwendung eines solchen schwermetallhaltigen Katalysators ist aber aus ökologischen Gründen nicht wünschenswert.

Aufgabe der vorliegenden Erfindung ist es, ein umweltverträgliches Verfahren zur Verfügung zu stellen, mit dem sich Fluor enthaltende Ethanderivate in guter Ausbeute und Selektivität in der Gasphase herstellen lassen. Diese Aufgabe wird durch das erfindungsgemäße Verfahren gelöst.

Das erfindungsgemäße Verfahren zur Herstellung von Fluor enthaltenden Ethanderivaten der allgemeinen Formel (I)

$CF_3\text{-}CHZ^1Z^2$   (I)

worin $Z^1$ und $Z^2$ gleich oder verschieden sein können und Wasserstoff, Fluor, Chlor oder Brom bedeuten, durch Umsetzung eines halogenierten Alkens oder halogenierten Alkans als Ausgangsverbindung mit Fluorwasserstoff in Anwesenheit eines Katalysators der allgemeinen Formel (II)

$AlCl_xF_y$   (II)

worin x 0 bis 1 bedeutet und y 3 bis 2 bedeutet, in der Gasphase bei einer Temperatur zwischen 250 und 450 °C, wobei man

a) als halogeniertes Alken eine Verbindung der allgemeinen Formel (III) verwendet

$F_kX_{2-k}C = CZ^1Z^2$   (III)

worin X Halogen außer Fluor ist, $Z^1$ und $Z^2$ die vorstehende Bedeutung besitzen und k 0, 1 oder 2 bedeutet, oder

b) als halogeniertes Alkan eine Verbindung der allgemeinen Formel (IV) verwendet

$F_kCl_{3-k}C\text{-}CHZ^1Z^2$   (IV)

worin k 0, 1 oder 2 bedeutet und $Z^1$ und $Z^2$ die vorstehende Bedeutung besitzen.

Die Verbindungen der Formel (I) enthalten immer mindestens ein Wasserstoffatom. Perhalogenierte Verbindungen und ihre Herstellung werden von der vorliegenden Erfindung nicht umfaßt.

Bevorzugt sind $Z^1$ und $Z^2$ gleich oder verschieden und bedeuten Wasserstoff, Fluor oder Chlor.

Besonders bevorzugt bedeutet $Z^1$ Wasserstoff und $Z^2$ Chlor.

So kann man nach dem erfindungsgemäßen Verfahren beispielsweise $CF_3CH_3$ aus Vinylidenfluorid, Vinylidenchlorid, Vinylidenchloridfluorid oder $CCl_3CH_3$, $CFCl_2CH_3$ oder $CF_2ClCH_3$ herstellen.

Das Verfahren der vorliegenden Erfindung eignet sich besonders zur Herstellung von $CF_3CH_2Cl$. Beispielsweise kann man $CF_3CH_2Cl$ aus Trihalogenethen wie $CF_2 = CHCl$ oder Tetrahalogenethanen herstellen.

Eine besonders bevorzugte Ausführungsform des erfindungsgemäßen Verfahrens ist dadurch gekennzeichnet daß man zur Herstellung von $CF_3CH_2Cl$

2

a) $CCl_2 = CHCl$ oder $CCl_2 = CCl_2$ mit Fluorwasserstoff umsetzt, wobei Fluorwasserstoff mindestens in derjenigen Menge eingesetzt wird, die stöchiometrisch zur Fluorwasserstoffanlagerung und zum Chlor-Fluor-Austausch am Alken notwendig ist, oder

b) $CCl_3CH_2Cl$, $CFCl_2CH_2Cl$, $CF_2ClCH_2Cl$ oder deren Gemische mit Fluorwasserstoff umsetzt, wobei Fluorwasserstoff mindestens in derjenigen Menge eingesetzt wird, die stöchiometrisch zum Chlor-Fluor-Austausch am Alkan notwendig ist.

Im erfindungsgemäßen Verfahren geht man bevorzugt von einem halogenierten Alken gemäß Variante a) aus. Zur Herstellung von $CF_3CH_2Cl$ geht man bevorzugt von $CCl_2 = CHCl$ aus.

Die vorliegende Erfindung wird nun anhand der bevorzugten Ausführungsform, nämlich der Herstellung von $CF_3CH_2Cl$, weiter erläutert.

Das erfindungsgemäße Verfahren kann bei vermindertem Druck, bei Umgebungsdruck oder erhöhtem Druck, beispielsweise bei bis zu 10 bar und höher, betrieben werden. Die Temperatur wird so gewählt, daß die Umsetzung in der Gasphase abläuft. Es hat sich als zweckmäßig erwiesen, die Umsetzung bei einer Temperatur von mindestens 250 °C durchzuführen. Man kann zwar auch unterhalb dieser Temperatur arbeiten, die Ausbeute ist dann jedoch geringer. Bevorzugt arbeitet man bei einem Druck, der dem Umgebungsdruck entspricht, also etwa 1 bar, bis hin zu einem Druck von etwa 5 bar absolut, insbesondere bei einem Druck von 1,5 bar absolut bis 4,5 bar absolut. Die Temperatur beträgt zweckmäßig zwischen etwa 250 und 400 °C, vorzugsweise zwischen 300 bis 350 °C.

Die im erfindungsgemäßen Verfahren erhaltenen Ethanderivate unterscheiden sich von den Ausgangsverbindungen dadurch, daß sie mindestens 1 Fluoratom mehr aufweisen. Für jedes auf diese Weise in das Substratmolekül eingeführte Fluoratom setzt man zweckmäßigerweise Fluorwasserstoff in einer Menge ein, die mindestens der stöchiometrisch notwendigen Menge entspricht. Die zur Fluorwasserstoff-Anlagerung bzw. zum Halogen-Fluor-Austausch eingesetzte Menge an Fluorwasserstoff kann auch größer sein. Sie kann beispielsweise das bis zu 15-fache und mehr der stöchiometrisch notwendigen Menge betragen. Bei der Herstellung von $CF_3CH_2Cl$ beispielsweise aus Trichlorethylen werden gute Ergebnisse erzielt, wenn die eingesetzte Menge an Fluorwasserstoff dem 1- bis 10-fachen, vorzugsweise dem 1- bis 6-fachen der stöchiometrisch notwendigen Menge entspricht.

Feuchtigkeit wirkt sich im erfindungsgemäßen Verfahren störend aus. Die Umsetzung wird daher zweckmäßigerweise unter Bedingungen durchgeführt, die verhindern, daß eine schädliche Menge an Wasser in die Reaktionsmischung gelangen kann. Man verwendet im wesentlichen wasserfreien Fluorwasserstoff. Handelsüblicher Fluorwasserstoff, der üblicherweise einen Wassergehalt von weniger als 0,1 Gew.-% aufweist, ist gut verwendbar. Weiterhin ist es empfehlenswert, die verwendete Apparatur in möglichst trockenem Zustand zu halten. Empfehlenswert ist es, Leitungen, Reaktionsgefäße und Einrichtungen zur Produktaufarbeitung und -aufbewahrung vor der Inbetriebnahme mit trockenen Gasen, beispielsweise mit trockener Luft oder trockenem Stickstoff, zu spülen. Zweckmäßig erwärmt man dabei die beheizbaren Teile der Apparatur auf mindestens 100 °C.

Gewünschtenfalls kann man das organische Ausgangsmaterial und/oder den eingesetzten Fluorwasserstoff mit Inertgas, beispielsweise trockenem Stickstoff, verdünnen.

Der Katalysator der allgemeinen Formel (II) kann zwar auf einen inerten Träger, beispielsweise Aktivkohle, aufgebracht verwendet werden. Bevorzugt wird jedoch auf einen Träger verzichtet, und der Katalysator der Formel (II) wird als Vollkontaktkatalysator eingesetzt.

Bei der Durchführung des erfindungsgemäßen Verfahrens können neben dem Katalysator der allgemeinen Formel (II) auch noch andere bekannte Katalysatoren in der Reaktionsmischung anwesend sein. Bevorzugt wird der Katalysator der Formel (II) als einziger Katalysator vorgesehen. Besonders bevorzugt verwendet man einen Katalysator der Formel (II), welcher Schwermetalle nicht oder allenfalls als geringfügige, herstellungsbedingte Beimengungen enthält.

In der Formel (II) für den Katalysator bedeuten x bevorzugt 0 bis 0,5, und y bevorzugt 3 bis 2,5.

Als Katalysator ist beispielsweise Aluminiumtrifluorid verwendbar. Bevorzugt verwendet man einen Katalysator der allgemeinen Formel (II), den man durch Umsetzung von im wesentlichen wasserfreien Aluminiumtrichlorid mit Fluorwasserstoff erhalten kann. Zweckmäßig arbeitet man bei der Durchführung dieser exothermen Reaktion bei einer Temperatur von bis zu 110 °C, um unerwünschtes Sublimieren von nicht umgesetztem Aluminiumtrichlorid zu vermeiden. Der Verlauf der Umsetzung zwischen Aluminiumtrichlorid und Fluorwasserstoff kann anhand der Menge des freigesetzten Chlorwasserstoffs verfolgt werden. Die Umsetzung kann abgebrochen werden, sobald die Temperatur im Reaktor trotz weiterer Fluorwasserstoffzufuhr abfällt. Gewünschtenfalls kann man den auf diese Weise erhältlichen Katalysator einer Nachbehandlung unterwerfen. Hierzu setzt man nach Beendigung der exothermen Reaktion zwischen Aluminiumtrichlorid und Fluorwasserstoff den Katalysator mit weiterem Fluorwasserstoff um. Dabei wird die Temperatur im Reaktor langsam auf bis zu 300 °C erhöht.

Bei der Herstellung des Katalysators ist die Anwesenheit von Wasser schädlich. Man verwendet deshalb wiederum im wesentlichen wasserfreien Fluorwasserstoff, beispielsweise handelsüblichen Fluorwasserstoff mit weniger als 0,1 Gew.-% Wasser. Den zur Umsetzung des Aluminiumchlorids benötigten Fluorwasserstoff kann man mit einem Inertgas, beispielsweise Stickstoff, verdünnen. Falls die Temperatur bei der exothermen Reaktion zwischen Aluminiumchlorid und Fluorwasserstoff zu stark ansteigt, kann man durch zusätzliche Verdünnung des verwendeten Fluorwasserstoff die Temperatur regeln.

Das Aluminiumchlorid wird zweckmäßig in Form grobkörniger Partikel eingesetzt. Bevorzugt verwendet man einen Katalysator, der in Form von Partikeln mit einem Durchmesser größer als 5 mm vorliegt. Zweckmäßig verwendet man Katalysatormaterial, dessen Partikel einen Durchmesser zwischen 5 mm und 20 mm aufweisen. Ein sehr gut geeignetes Granulat ist von der Firma BASF erhältlich.

Gewünschtenfalls kann der Katalysator in situ bei der Umsetzung zwischen dem organischen Ausgangsmaterial und Fluorwasserstoff hergestellt werden. Zweckmäßig stellt man den Katalysator jedoch in einer vorgeschalteten Verfahrensstufe her.

Das organische Ausgangsmaterial bzw. der Fluorwasserstoff werden zweckmäßig verdampft und dann über den Katalysator geleitet. Gewünschtenfalls kann man das organische Material und den Fluorwasserstoff vor oder nach dem Verdampfen vermischen und dann vorgemischt über den Katalysator leiten.

Die Aufarbeitung des Reaktionsgemisches erfolgt in üblicher Weise. Zunächst leitet man das Reaktionsgemisch durch einen Gaswäscher, der eine geeignete Waschflüssigkeit zur Abtrennung von Fluorwasserstoff und Chlorwasserstoff, beispielsweise Wasser, enthält. Die den Gaswäscher verlassenden Reaktionsprodukte werden zweckmäßig kondensiert und anschließend fraktioniert destilliert. Nebenprodukte wie nicht vollständig fluorierte Verbindungen sowie etwaig vorhandenes Ausgangsmaterial kann in den Reaktor zurückgeführt werden.

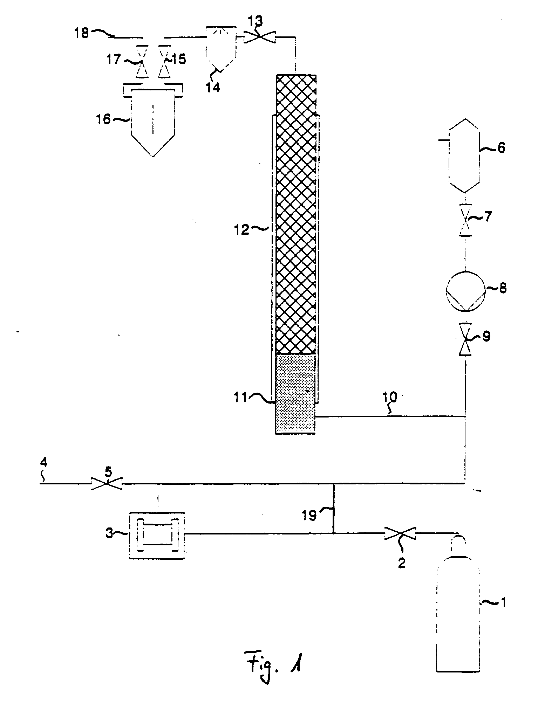

Das erfindungsgemäße Verfahren wird nun in Verbindung mit Fig.1, die eine für das erfindungsgemäße Verfahren verwendbare zweckmäßige Apparatur beschreibt, weiter erläutert.

Die für die Durchführung verwendete Apparatur sollte gegenüber Fluorwasserstoff, Chlorwasserstoff, Aluminiumhalogeniden und dem jeweils verwendeten organischen Ausgangsmaterial resistent sein. Zweckmäßig verwendet man Bauteile aus Teflon und speziellen Fluorwasserstoff-resistenten Legierungen, beispielsweise Nickellegierungen wie "Hastelloy".

Fluorwasserstoff wird dem Vorratsbehälter 1 entnommen und über das Ventil 2 in den Fluorwasserstoff-Verdampfer 3 eingeleitet. Über die Inertgasleitung 4 und das Ventil 5 kann in den erhitzten Fluorwasserstoff gewünschtenfalls Inertgas zugemischt werden. Der gegebenenfalls mit Inertgas verdünnte Fluorwasserstoff wird dann mit Trichlorethylen vermischt, welches aus dem Vorratsbehälter 6 über ein Ventil 7, eine Dosierpumpe 8 und ein weiteres Ventil 9 entnommen wird. Das vermischte Ausgangsmaterial wird dann über die Leitung 10 in den Vorverdampfer 11 geleitet, dort erhitzt und dann in den Reaktor 12 geleitet. Im Reaktor reagiert das Gemisch mit zuvor hergestelltem Katalysator der Formel (II). Über das Ventil 13 wird das Reaktionsgemisch zunächst einem Abscheider 14 zugeführt. Dort werden mitgerissene flüssige oder feste Bestandteile aus dem Reaktionsprodukt abgeschieden. Das Reaktionsgemisch wird sodann über das Ventil 15 in den Kühler 16 geleitet und aus diesem über das Ventil 17 und die Leitung 18 einem mit Wasser gefüllten Gaswäscher zugeleitet. Das aus dem in Figur 1 nicht mehr abgebildeten Gaswäscher austretende Reaktionsgemisch wird dann kondensiert und fraktioniert destilliert. Der Fluorwasserstoffverdampfer 3, der Vorverdampfer 11 und der Reaktor 12 sind thermostatisierbar, beispielsweise mittels einem Wärmeträgermedium wie Thermoöl. Der Fluorwasserstoffverdampfer 3 kann durch die Bypass-Leitung 19 umgangen werden.

Die durch das erfindungsgemäße Verfahren erhaltenen fluorhaltigen Ethanderivate sind wertvolle umweltverträgliche Lösungsmittel, Treibmittel sowie Zwischenprodukte für die chemische Synthese.

Das erfindungsgemäße Verfahren zeichnet sich durch hohen Umsatz, hohe Selektivität und hohe Umweltverträglichkeit aus.

Die folgenden Beispiele sollen das erfindungsgemäße Verfahren weiter erläutern, ohne es in seinem Umfang einzuschränken.

Beispiel 1:

Herstellung von 1.1.1.-Trifluor-2-chlorethan (R133a)

1.1. Verwendete Apparatur

Die verwendete Apparatur entsprach der in Figur 1 beschriebenen Apparatur. Ein Fluorwasserstoff-Vorratsbehälter war über eine Leitung mit einem Fluorwasserstoff-Verdampfer verbunden. Über eine absperrbare Stickstoff-Leitung konnte Stickstoff und über eine weitere absperrbare Leitung Trichlorethylen aus einem Vorratsbehälter in die Fluorwasserstoff-Leitung eingeleitet werden. Die Fluorwasserstoff-Leitung endete in einem Vorverdampfer, der mit einer Heizvorrichtung (in Heizrohren zirkulierendes Thermoöl aus einem Thermostaten) versehen war. Der Vorverdampfer war mit einem aus Edelstahl gefertigten Doppelmantel-Reaktor verbunden. Das innere Rohr (Durchmesser 36 mm) diente als Reaktorkammer. Durch den Raum zwischen innerem und äußerem Rohr wurde über entsprechende Anschlüsse Thermoöl mit Hilfe eines Thermostaten durchgeleitet (alternativ könnte man auch einen elektrisch beheizbaren Reaktor vorsehen). Das dem Einlaß der Ausgangsverbindungen entgegengesetzte Ende des Reaktors war mit einer Sicherheitswaschflasche und diese mit einem Wäscher verbunden. Der Wäscher war mit Wasser gefüllt. Der Wäscher war mit einer Tiefkühlfalle verbunden. Die Tiefkühlfalle wiederum war mit einer Destillationsapparatur verbunden.

1.2. Herstellung des Katalysators

Zunächst wurde die Apparatur mit getrocknetem Stickstoff gespült. In das innere Rohr des Edelstahl-Doppelmantelreaktors wurden 600 g wasserfreies Aluminiumchlorid einer Partikelgröße zwischen 1 und 2 cm (Hersteller: BASF, Ludwigshafen) eingefüllt. Die Leitung zur Zuführung von Trichlorethylen blieb geschlossen. Die Leitung zur Zuführung von Fluorwasserstoff wurde geöffnet und das resultierende Gemisch von Fluorwasserstoff und Stickstoff wurde über den Vorverdampfer in den Reaktor eingeleitet. Die Volumenströme wurden so eingeregelt, daß eine Temperatur im Reaktor zwischen etwa 50 °C und 70 °C aufrecht erhalten wurde. Es wurde darauf geachtet, daß die Temperatur nicht über etwa 110 °C anstieg. Nach Beendigung der exothermen Fluorierung des Aluminiumchlorids wurden im Wäscher etwa 75 % der Chloratome in Form von HCl vorgefunden. Der erhaltene Katalysator entsprach demnach annähernd einer Verbindung der Bruttoformel $AlCl_{0,75}F_{2,25}$. Der hergestellte Katalysator wurde einer Nachbehandlung unterworfen, indem durch den Reaktor unter kontinuierlicher Temperaturerhöhung und weiterer Dosierung eines Fluorwasserstoff-Stickstoff-Gemisches die Temperatur auf etwa 300 °C gebracht wurde. Nach dem Abkühlen wurde dem Material eine erneute Probe entnommen und analysiert. Im nachbehandelten katalytischen Material war nahezu das gesamte Chlor gegen Fluor ausgetauscht. Der Katalysator enthielt gemäß einer Elementaranalyse nur noch 0,007 Gew.-% Chlor. Die Ausbeute war quantitativ.

1.3. Herstellung von 1.1.1.-Trifluor-2-Chlorethan aus Trichlorethylen und Fluorwasserstoff in Anwesenheit des gemäß 1.2. hergestellten Katalysators

Der Vorverdampfer wurde auf eine Temperatur von etwa 150 °C gebracht. Der Reaktor, der den gemäß Beispiel 1.2. hergestellten Katalysator enthielt, wurde auf 303 bis 306 °C temperiert. In kontinuierlicher Verfahrensweise wurde 2,5 Stunden lang ein Gemisch von Fluorwasserstoff, welcher mit Stickstoff verdünnt worden war, und Trichlorethylen zunächst in den Vorverdampfer eingeleitet und dort in den Gaszustand überführt. Das Gemisch wurde dann im Reaktor unter Kontaktierung mit dem Katalysator umgesetzt. Pro Stunde wurden 1,2 mol (25,0 g) Fluorwasserstoff und 0,4 mol (36 ml) Trichlorethylen durch den Reaktor geleitet. Das Molverhältnis von Fluorwasserstoff zu Trichlorethylen betrug somit 3:1. Der Druck schwankte während der Umsetzung zwischen 1,7 und 3,9 bar absolut (0,7 und 2,9 bar Überdruck). Die Ursache für diese Druckschwankungen war in einem unterschiedlichen Fluorwasserstoff-Vordruck und nicht genau feinregulierbaren Ventilen zu suchen. Das aus dem Reaktor abgeführte Rohprodukt wurde über die Sicherheitswaschflasche in den Wäscher geführt, in welchem Chlorwasserstoff und Fluorwasserstoff ausgewaschen wurden. Den Wäscher passieren im wesentlichen nur noch organische Bestandteile sowie der Stickstoff. Nach Kondensation der organischen Bestandteile in der Tiefkühlfalle wurden diese fraktioniert destilliert.

Dem Rohprodukt wurden nach Passieren des Wäschers nach 90 Minuten, nach 120 Minuten sowie nach 150 Minuten Proben entnommen, die gaschromatographisch untersucht wurden. Die Gewichtsanteile bestimmter Verbindungen im Rohprodukt sowie die Gewichtsanteile bestimmter Verbindungen in der organischen Phase nach Abzug des enthaltenen Stickstoffes sind in Tabelle 1 zusammengestellt. Der Tabelle läßt sich entnehmen, daß bereits bei Einsatz der stöchiometrisch notwendigen Fluorwasserstoffmenge die Ausbeute schon zu Beginn der Umsetzung gut ist und im Verlauf der Umsetzung sogar zunimmt.

## Tabelle 1 (Beispiel 1.3)

| Analyse: Probenentnahmen nach [min]: | | | | | | |
|---|---|---|---|---|---|---|
| | 90 Anteil in der Probe | | 120 Anteil in der Probe | | 150 Anteil in der Probe | |
| Bestandteil | [Gew.-%] | 1) | [Gew.-%] | 1) | [Gew.-%] | 1) |
| Stickstoff | 62,2 | - | 68,4 | - | 89,0 | - |
| R 133a | 20,4 | 56 | 17,0 | 56,1 | 7,3 | 75,3 |
| Tri* | 6,5 | 17,9 | 7,9 | 26 | 0,9 | 9,3 |
| andere: | | | | | | |
| R 113 | 3,3 | 9,1 | 3,1 | 10,2 | 0,9 | 9,3 |
| R 132 | 3,8 | 10,4 | 1,5 | 5 | 0,6 | 6,2 |
| R 143 | 2,2 | 6 | 0,5 | 1,7 | | |
| R 1121a | 0,2 | 0,5 | 0,3 | 1 | | |

1) bezogen auf den Gehalt an organischen Bestandteilen in der gezogenen Probe nach Abzug des Gehaltes an Stickstoff, Angaben in [Gew.%]

\* Trichlorethylen

Beispiel 2:

Beispiel 1.3 wurde wiederholt. Als Katalysator wurde der bereits in Beispiel 1.3. verwendete Katalysator eingesetzt. Der Vorverdampfer wurde wiederum auf etwa 150 °C eingeregelt, die Temperatur im Reaktor auf 300 bis 310 °C. Der Druck schwankte zwischen 1,0 und 2,1 bar Überdruck. Über eine Zeitdauer von 330 Minuten wurde dann kontinuierlich ein Gemisch von Stickstoff, Fluorwasserstoff und Trichlorethylen in den Reaktor eingeleitet. Pro Stunde wurden 1,95 mol (39,1 g) Fluorwasserstoff und 0,38 mol (34,5 ml) Trichlorethylen eingesetzt. Das Molverhältnis von Fluorwasserstoff zu Trichlorethylen betrug etwa 5,1:1. Die Aufarbeitung des Rohproduktes erfolgte wie in 1.3. beschrieben. Dem Rohprodukt wurden nach Verlassen des Wäschers nach 120 Minuten, 210 Minuten, 270 Minuten sowie 330 Minuten Proben entnommen, die gaschromatographisch untersucht wurden. Die Ergebnisse sind in Tabelle 2 wiedergegeben. Der Anteil an R133a im Rohprodukt nimmt bei Einsatz einer überstöchiometrischen Menge an Fluorwasserstoff zu.

## Tabelle 2 (Beispiel 2)

| Analyse: Probenentnahmen nach [min]: | 120 | | 210 | | 270 | | 330 | |
|---|---|---|---|---|---|---|---|---|
| Bestand-teil | Gehalt in der Probe [Gew.-%] | 1) | Gehalt in der Probe [Gew.-%] | 1) | Gehalt in der Probe [Gew.-%] | 1) | Gehalt in der Probe [Gew.-%] | 1) |
| Stickstoff | 69,9 | – | 43,4 | – | 44,9 | – | 48,8 | – |
| R 133a | 18,2 | 67,2 | 45,2 | 82,3 | 43,7 | 81,8 | 40,9 | 82,3 |
| Tri* | 4,9 | 18,1 | 4,3 | 7,8 | 4,1 | 7,7 | 3,6 | 7,2 |
| andere: | | | | | | | | |
| R 113 | 0,9 | 3,3 | 1,7 | 3,1 | 1,7 | 3,2 | 1,7 | 3,4 |
| R 132 | 2,7 | 10 | 1,8 | 3,3 | 1,6 | 3 | 1,4 | 2,8 |
| R 143 | | | 1,9 | 3,5 | 2,3 | 4,3 | 2,1 | 4,2 |
| Tetra** | 0,4 | 1,5 | | | | | | |

1) bezogen auf den Gehalt an organischen Bestandteilen in der gezogenen Probe nach Abzug des Gehalts an Stickstoff, Angaben in [Gew.-%]

\* Trichlorethylen

\*\* Tetrachlorethylen

Beispiel 3:

Die Vorgehensweise ensprach der in Beispiel 1.3. beschriebenen. Die Temperatur im Vorverdampfer wurde auf 149 °C eingestellt. Die Temperatur des Reaktors wurde geringfügig erhöht, nämlich auf 317 bis 334 °C. Der Druck betrug zwischen 1,8 und 3,1 bar Überdruck. Während einer Zeitdauer von 360 Minuten wurde kontinuierlich ein Gemisch von Stickstoff, Fluorwasserstoff und Trichlorethylen über den Vorverdampfer in den Reaktor eingeleitet und in Anwesenheit des Katalysators umgesetzt. Pro Stunde wurden 2,79 mol (55,8 g) Fluorwasserstoff und 0,54 mol (48,3 ml) Trichlorethylen eingesetzt. Das Molverhältnis von Fluorwasserstoff zu Trichlorethylen betrug etwa 5,2:1. Wie der Tabelle 3 zu entnehmen ist, ermöglicht die geringfügige Erhöhung der Temperatur den Durchsatz größerer Mengen an Ausgangsverbindungen bei gleichem Umsatzgrad.

Tabelle 3: (Beispiel 3)

| Analyse: Probenentnahmen nach [min]: | 91 | | 150 | | 210 | |
|---|---|---|---|---|---|---|
| Bestand-teil | Gehalt in der Probe [Gew.-%] | 1) | Gehalt in der Probe [Gew.-%] | 1) | Gehalt in der Probe [Gew.-%] | 1) |
| Stickstoff | 66,1 | -- | 58,0 | -- | 24,9 | -- |
| 133a | 20,7 | 67 | 30,9 | 79,8 | 62,8 | 84,6 |
| Tri* | 6,3 | 20,4 | 4,8 | 12,4 | 4,7 | 6,3 |
| andere: | | | | | | |
| 113 | 1,1 | 3,6 | 1,5 | 3,9 | 2,8 | 3,8 |
| 132 | 2,3 | 7,4 | 1,3 | 3,4 | 0,9 | 1,2 |
| 134a | | | | | 0,2 | 0,3 |
| (H2O) (143) | | | | | 2,7 | 3,6 |
| Tetra** | 0,5 | 1,6 | 0,2 | 0,5 | 0,1 | 1,3 |

Fortsetzung Tabelle 3:

| Analyse: Probeentnahmen nach [min]: | 270 | | 330 | | 360 | |
|---|---|---|---|---|---|---|
| Bestand-teil | Gehalt in der Probe [Gew.-%] | 1) | Gehalt in der Probe [Gew.-%] | 1) | Gehalt in der Probe [Gew.-%] | 1) |
| Stickstoff | 18,2 | -- | 69,1 | -- | 25,8 | -- |
| R 133a | 70,4 | 87,6 | 22,0 | 72,8 | 60,1 | 82,8 |
| Tri* | 2,5 | 3,1 | 4,8 | 15,9 | 4,6 | 6,3 |
| andere: | | | | | | |
| R 113 | 2,4 | 3 | 1,3 | 4,3 | 2,6 | 3,6 |
| R 132 | 0,8 | 1 | 0,4 | 1,3 | 0,9 | 1,2 |
| R 134a | 0,2 | 0,2 | 0,34 | 1,1 | 0,2 | 0,2 |
| R 143 | 4,0 | 5 | 1,1 | 3,6 | 4,1 | 5,6 |
| Tetra** | 0,1 | 0,1 | 0,3 | 1 | 0,1 | 1,4 |

1) bezogen auf den Gehalt an organischen Bestandteilen in der gezogenen Probe nach Abzug des Gehalts an Stickstoff, Angaben in [Gew.-%].

\* Trichlorethylen

\*\* Tetrachlorethylen

**Patentansprüche**

1. Verfahren zur Herstellung von Fluor enthaltenden Ethanderivaten der allgemeinen Formel (I)

$CF_3\text{-}CHZ^1Z^2$   (I)

worin $Z^1$ und $Z^2$ gleich oder verschieden sein können und Wasserstoff, Fluor, Chlor oder Brom bedeuten, durch Umsetzung eines halogenierten Alkens oder halogenierten Alkans als Ausgangsverbindung mit Fluorwasserstoff in Anwesenheit eines Katalysators der allgemeinen Formel (II)

$$AlCl_xF_y \quad (II)$$

worin x 0 bis 1 bedeutet und y 3 bis 2 bedeutet, in der Gasphase bei einer Temperatur zwischen 250 und 450 °C, wobei man

a) als halogeniertes Alken eine Verbindung der allgemeinen Formel (III) verwendet

$$F_kX_{2-k}C = CZ^1Z^2 \quad (III)$$

worin X Halogen außer Fluor ist, $Z^1$ und $Z^2$ die vorstehende Bedeutung besitzen und k 0, 1 oder 2 bedeutet, oder

b) als halogeniertes Alkan eine Verbindung der allgemeinen Formel (IV) verwendet

$$F_kCl_{3-k}C\text{-}CHZ^1Z^2 \quad (IV)$$

worin k 0, 1 oder 2 bedeutet und $Z^1$ und $Z^2$ die vorstehende Bedeutung besitzen.

2. Verfahren nach Anspruch 1, dadurch gekennzeichnet, daß $Z^1$ und $Z^2$ gleich oder verschieden sein können und Wasserstoff, Fluor oder Chlor bedeuten.

3. Verfahren nach Anspruch 1 oder 2, dadurch gekennzeichnet, daß $Z^1$ Wasserstoff und $Z^2$ Chlor bedeuten.

4. Verfahren nach einem der vorhergehenden Ansprüche, dadurch gekennzeichnet, daß man zur Herstellung von $CF_3CH_2Cl$
a) $CCl_2 = CHCl$ oder $CCl_2 = CCl_2$ mit Fluorwasserstoff umsetzt, wobei Fluorwasserstoff mindestens in derjenigen Menge eingesetzt wird, die stöchiometrisch zur Fluorwasserstoffanlagerung und zum Chlor-Fluor-Austausch am Alken notwendig ist, oder
b) $CCl_3CH_2Cl$, $CFCl_2CH_2Cl$, $CF_2ClCH_2Cl$ oder deren Gemische mit Fluorwasserstoff umsetzt, wobei Fluorwasserstoff mindestens in derjenigen Menge eingesetzt wird, die stöchiometrisch zum Chlor-Fluor-Austausch am Alkan notwendig ist.

5. Verfahren nach einem der vorhergehenden Ansprüche, dadurch gekennzeichnet, daß man von einem halogenierten Alken ausgeht.

6. Verfahren nach Anspruch 4 oder 5, dadurch gekennzeichnet, daß man zur Herstellung von $CF_3CH_2Cl$ ausgeht von $CCl_2 = CHCl$.

7. Verfahren nach einem der vorhergehenden Ansprüche, dadurch gekennzeichnet, daß man bei einem Druck von 1 bis 5 bar absolut, vorzugsweise bei einem Druck von 1,5 bis 4,5 bar absolut arbeitet.

8. Verfahren nach einem der vorhergehenden Ansprüche, dadurch gekennzeichnet, daß man bei einer Temperatur zwischen etwa 250 und 400 °C arbeitet, vorzugsweise bei 300 bis 350 °C.

9. Verfahren nach einem der vorhergehenden Ansprüche, dadurch gekennzeichnet, daß man einen Katalysator der allgemeinen Formel (II) verwendet, der erhalten wird durch Umsetzung von $AlCl_3$ mit Fluorwasserstoff bei einer Temperatur von bis zu 110 °C, wobei man gewünschtenfalls den hergestellten Katalysator einer Nachbehandlung unterwirft.

10. Verfahren nach Anspruch 9, dadurch gekennzeichnet, daß man den Katalysator einer Nachbehandlung unterwirft, indem man nach Beendigung einer exothermen Reaktion den gebildeten Katalysator mit weiterem Fluorwasserstoff unter Temperaturerhöhung auf bis zu 300 °C umsetzt.

## Claims

1. A process for preparing fluorine-containing ethane derivatives of the general Formula (I),

EP 0 492 386 B1

$$CF_3\text{-}CHZ^1Z^2 \quad (I)$$

wherein $Z^1$ and $Z^2$ may be identical or different and stand for hydrogen, fluorine, chlorine or bromine, by reacting a halogenated alkene or halogenated alkane as a starting compound with hydrogen fluoride in the presence of a catalyst of the general Formula (II),

$$AlCl_xF_y \quad (II)$$

wherein x is 0 to 1 and y is 3 to 2, in the gas phase at a temperature of between 250 and 450°C, in which

a) a compound of the general Formula (III),

$$F_kX_{2-k}C = CZ^1Z^2 \quad (III)$$

wherein X is halogen with the exception of fluorine, $Z^1$ and $Z^2$ have the above meanings and k is 0, 1 or 2,
is used as the halogenated alkene, or
b) a compound of the general Formula (IV),

$$F_kCl_{3-k}C\text{-}CHZ^1Z^2 \quad (IV)$$

wherein k is 0, 1 or 2 and $Z^1$ and $Z^2$ have the above meanings,
is used as the halogenated alkane.

2. A process according to Claim 1, characterised in that $Z^1$ and $Z^2$ may be identical or different and stand for hydrogen, fluorine or chlorine.

3. A process according to Claim 1 or 2, characterised in that $Z^1$ is hydrogen and $Z^2$ is chlorine.

4. A process according to one of the preceding Claims, characterised in that in order to prepare $CF_3CH_2Cl$
a) $CCl_2 = CHCl$ or $CCl_2 = CCl_2$ is reacted with hydrogen fluoride, with hydrogen fluoride being used at least in a quantity which is stoichiometrically necessary for the addition of hydrogen fluoride and for the chlorine-fluorine exchange on the alkene, or
b) $CCl_3CH_2Cl$, $CFCl_2CH_2Cl$, $CF_2ClCH_2Cl$ or mixtures thereof are reacted with hydrogen fluoride, with hydrogen fluoride being used at least in a quantity which is stoichiometrically necessary for the chlorinefluorine exchange on the alkane.

5. A process according to one of the preceding Claims, characterised in that the starting point is a halogenated alkene.

6. A process according to Claim 4 or 5, characterised in that for the production of $CF_3CH_2Cl$ the starting point is $CCl_2 = CHCl$.

7. A process according to one of the preceding Claims, characterised in that one operates at a pressure of 1 to 5 bar absolute, preferably at a pressure of 1.5 to 4.5 bar absolute.

8. A process according to one of the preceding Claims, characterised in that one operates at a temperature between about 250 and 400°C, preferably at 300 to 350°C.

9. A process according to one of the preceding Claims, characterised in that a catalyst of the general formula (II) is used, which is obtained by reacting $AlCl_3$ with hydrogen fluoride at a temperature of up to 110°C, and if desired the catalyst produced is subjected to subsequent treatment.

10. A process according to Claim 9, characterised in that the catalyst is subjected to subsequent treatment in that once an exothermic reaction has ended the resulting catalyst is reacted with further hydrogen fluoride with a temperature increase to up to 300°C.

10

**Revendications**

1. Procédé de préparation de dérivés fluorés de l'éthane, de formule générale (I)

   $$CF_3 - CHZ^1Z^2 \quad (I)$$

   dans laquelle $Z^1$ et $Z^2$ peuvent être identiques ou différents et ils représentent chacun un atome d'hydrogène, de fluor, de chlore ou de brome par réaction d'un alcène halogéné ou d'un alcane halogéné, comme composé de départ, avec le fluorure d'hydrogène en présence d'un catalyseur de formule générale (II)

   $$AlCl_xF_y \quad (II)$$

   dans laquelle x vaut 0 à 1 et y vaut 3 à 2 en phase gazeuse à une température comprise entre 250 et 450°C, procédé dans lequel on utilise :
   (a) Comme alcène halogéné, un composé de formule générale (III)

   $$F_kX_{2-k}C = CZ^1Z^2 \quad (III)$$

   dans laquelle X représente un atome d'halogène autre que le fluor; $Z^1$ et $Z^2$ ont le sens précité; et k vaut 0, 1 ou 2 ou
   (b) Comme alcane halogéné, un composé de formule générale (IV)

   $$F_kCl_{3-k}C-CHZ^1Z^2 \quad (IV)$$

   dans laquelle k vaut 0, 1 ou 2 et $Z^1$ et $Z^2$ ont le sens précité.

2. Procédé selon la revendication 1, caractérisé en ce que $Z^1$ et $Z^2$ peuvent être identiques ou différents et ils représentent chacun un atome d'hydrogène, de fluor ou de chlore.

3. Procédé selon la revendication 1 ou 2, caractérisé en ce que $Z^1$ représente un atome d'hydrogène et $Z^2$ représente un atome de chlore.

4. Procédé selon l'une des revendications précédentes, caractérisé en ce que, pour la préparation de $CF_3CH_2Cl$ :
   (a) On fait réagir $CCl_2 = CHCl$ ou $CCl_2 = CCl_2$ avec le fluorure d'hydrogène, le fluorure d'hydrogène étant utilisé au moins en quantité stoéchiométriquement nécessaire pour la fixation du fluorure d'hydrogène et pour le remplacement chlore-fluor sur l'alcène, ou
   (b) on fait réagir $CCl_3CH_2Cl$, $CFCl_2CH_2Cl$, $CF_2ClCH_2Cl$ ou leurs mélanges avec le fluorure d'hydrogène, en utilisant le fluorure au moins en quantité stoéchiométriquement nécessaire pour le remplacement chlore-fluor sur l'alcane.

5. Procédé selon l'une des revendications précédentes, caractérisé en ce qu'on part d'un alcène halogéné.

6. Procédé selon la revendication 4 ou 5, caractérisé en ce que, pour préparer $CF_3CH_2Cl$, on part de $CCl_2 = CHCl$.

7. Procédé selon l'une des revendications précédentes, caractérisé en ce qu'on travaille sous une pression absolue de 1 à 5 bars, avantageusement sous une pression absolue de 1,5 à 4,5 bars.

8. Procédé selon l'une des revendications précédentes, caractérisé en ce qu'on travaille à une température comprise entre 250 et 400°C, avantageusement à 300 jusqu'à 350°C.

9. Procédé selon l'une des revendications précédentes, caractérisé en ce qu'on utilise un catalyseur de formule générale (II), que l'on obtient par la réaction de $AlCl_3$ avec du fluorure d'hydrogène à une température allant jusqu'à 110°C, en soumettant, si on le souhaite, à un post-traitement le catalyseur ainsi préparé.

**10.** Procédé selon la revendication 9, caractérisé en ce qu'on soumet le catalyseur à un post-traitement en faisant réagir, après achèvement d'une réaction exothermique, le catalyseur formé avec un supplément de fluorure d'hydrogène en élevant la température jusqu'à 300 ° C.

Fig. 1